# EUROPEAN PATENT APPLICATION

(11) **EP 4 575 460 A1**
(43) Date of publication of application: **25.06.2025**
(21) Application number: 24221184.5
(22) Date of filing: 18.12.2024
(51) Int. Cl.: G01N 15/1433, G01N 15/14

(54) **A DEVICE AND METHOD FOR CHARACTERIZATION OF BIOLOGICAL CELLS**

(30) Priority: 22.12.2023 EP 23219764
(71) Applicant: Imec VZW, 3001 Leuven (BE)
(72) Inventor: VAN ROY, Willem, 3360 Bierbeek (BE)
(74) Representative: AWA Sweden AB

(57) **Abstract**

According to an aspect of the present inventive concept there is provided a device for characterizing biological cells comprising: a flow channel passing a flow of liquid carrying the biological cells to be characterized, the flow channel comprises at least one zone associated with a cell category; at least one detector to detect information representing the biological cells passing the at least one zone; a processor configured to receive the information and to process the information by: dividing each zone into segments; identifying candidate biological cells; identifying tracks from each of the candidate biological cells; for each track, determining a set of transit times, wherein each transit time represents a transit time of the candidate biological cell through a segment; for each candidate biological cell, processing the set of transit times for characterizing the candidate biological cell by determining whether the candidate biological cell is a biological cell belonging to an associated biological cell category of each respective zone.

## Description

### Technical field

The present description relates to a device for characterizing biological cells and a method for characterizing biological cells.

### Background

Characterization of biological cells is important for cell analysis and research. The characterization may be used to find similarities or differences between cells. It may further be used for cell sorting. Today, cells are commonly characterized by biomarkers on the surface of the cell membrane. This may be made by labeling the cells. For instance, biomarkers on the cells may be marked using antibodies connected to fluorescent molecules. However, the labeling may be invasive, and a further use of the cell may be difficult. Thus, there is a need for improvements of the field.

### Summary

An objective of the present description is to enable characterization of biological cells. It is a further objective of the present invention to enable label free characterization of biological cell, allowing for a high throughput characterization. These and other objectives of the invention are at least partly met by the invention as defined in the independent claims. Preferred embodiments are set out in the dependent claims. In the following the term cell refers to a biological cell, unless otherwise stated.

According to a first aspect, there is provided a device for characterizing biological cells comprising:
a flow channel configured to pass a flow of liquid, wherein:
   the flow of liquid carries the biological cells to be characterized,
   the flow channel comprises at least one zone,
   each zone of the flow channel is associated with a cell category, and
   each zone of the flow channel comprises at least one surface coated with molecules having an affinity and specificity to the cell category associated with the zone, the at least one surface of the zone being configured to modify, by the molecules coating the at least one surface, a movement of a biological cell belonging to an associated biological cell category of the zone as the cell, carried by the flow of liquid, passes the zone, whereby the at least one surface forms at least one cell movement modifying (CMM) surface, and whereby the modified movement of the biological cell caused by the CMM surface forms a specific modified movement;
at least one detector configured to detect information representing the biological cells, carried by the flow of liquid, passing the at least one zone;
a processor configured to receive the information detected by the at least one detector, and to process the information, wherein processing of the information comprises:
   dividing each zone of the flow channel into segments;
   identifying candidate biological cells;
   identifying tracks from each of the candidate biological cells;
   for each track of the candidate biological cells, determining a set of transit times, wherein each transit time in the set represents a transit time of the candidate biological cell through a segment;
   for each candidate biological cell, processing the set of transit times for characterizing the candidate biological cell.

It is a realization of the inventors that the device provides with an easy and robust characterization of biological cells. The device allows for a characterization of cell depending on several parameters.

The device is configured to cause movement of biological cells to be affected by the CMM surface. Thus, a track of the biological cell is affected by modified movements caused by the CMM surface, such that the track may hold information of cell properties, i.e. characterization, of the biological cells.

The characterization performed by the device may be used as a digital biomarker of the biological cells. In other words, without labeling the biological cells the device may classify the cells into groups and the device thus gives information about the cells with a non-invasive technique.

Cells may be characterized by the molecules they comprise, e.g. the molecules comprised in the cell membrane. Molecules comprised in the cell membrane may e.g. be antigens. A way of checking whether a cell comprises a certain cell membrane molecule may be to let the cell interact with a molecule that binds specifically to said cell membrane molecule. For example, an antigen may correspond to one or more antibodies, where the antigen and antibody bind specifically to each other. Consequently, cells may be assigned into cell categories depending on which cell membrane molecules they comprise or which molecules the cells bind to. For example, the antigen CD4 (cluster of differentiation 4) molecule in cell membranes is a glycoprotein which binds to anti-CD4 antibodies (CD4 positive cells). Cells that bind to anti-CD4 antibodies may form one cell category. Such cells may be characterized by their modified movements when they travel through a zone with a CMM surface coated by anti-CD4 antibodies. Analogously, cells that bind to anti-CD8 (cluster of differentiation 8) antibodies (CD8 positive cells) may form another cell category. Such cells may be characterized by their modified movements when they travel through a zone with a CMM surface coated by anti-CD8 antibodies. Thus, a cell category may be a category off cells which binds to a specific molecule such as an antibody.

A biological cell may be assigned to more than one cell category. In other words, the cell membrane of a cell may have antigens against several molecules. A cell may for instance be both CD4 positive and CD8 positive. There are several antigens on a cell membrane and a combination of antibodies, or other biomarkers, against those antigens may be used to categorize cells within a larger group of cells. As an example, in a cell line the cells comprised in the cell line may belong to one, two, three, four or even more cell categories. Cell categories may also overlap, such that some of the cells belonging to a first cell category may have another marker belonging to a second cell category. The second cell category may comprise of cells belonging to the first category and other cells belonging to a third cell category.

Characterizing should be interpreted such that the device acquires information describing the cell in a general manner. This information may be used to classify the cell into different cell categories. However, it is not limited to a classification but may be a more general information acquirement. Throughout the application, it should be understood that by characterization the type of classification into the cell category is only one example of how the information may be used.

The device may comprise more than one zone, wherein each zone may be coated with molecules having an affinity and a specificity to a respective cell category. Then, biological cells passing the multiple zones may interact with none, one or several of the CMM surfaces depending on the biomarkers on the surface of the biological cell.

For a proper classification and a possible further analysis of the CD4 positive cells, the cell behavior when passing over the CMM surface may be combined with other properties of the biological cell to classify it.

The molecules coating at least one CMM surface of the at least one zone of the flow channel may comprise at least one of: antibodies, aptamers, antibody fragments, peptides, molecularly imprinted polymers, receptors or lectins. Antibodies, aptamers, antibody fragments, peptides, molecularly imprinted polymers, receptors or lectins may have an affinity to specific cell categories but not to other cell categories. Antibodies, aptamers, antibody fragments, peptides, molecularly imprinted polymers, receptors or lectins may bind to specific cell categories but not to other cell categories. Antibodies, aptamers, antibody fragments, peptides, molecularly imprinted polymers, receptors or lectins may have a high sensitivity in binding to a cell category. Thus, the percentage of true positive binding events may be high. Antibodies, aptamers, antibody fragments, peptides, molecularly imprinted polymers, receptors or lectins may have a high specificity in binding to a cell category. Thus, the percentage of false positive binding events may be low. Alternatively, the molecules coating at least one CMM surface of the at least one zone of the flow channel may comprise other molecules than antibodies, aptamers, and lectins, e.g. comprise other molecules that binds specifically to a cell membrane molecule.

A CMM surface may be configured to modify the movement of the cell belonging to the associated cell category of the zone by modifying e.g. the speed, direction or path of cells comprised in the cell category. For example, the at least one CMM surface of at least one zone of the flow channel may be configured to modify the movement of the cell belonging to the associated cell category of the zone by slowing down the speed of the cell movement. This may be made by the molecules binding and releasing to the cell belonging to the cell category such that the speed slows down. As a second example, the at least one CMM surface of at least one zone of the flow channel may be configured to modify the movement of the cell belonging to the associated cell category of the zone by binding and releasing the cell belonging to the associated cell category of the zone. Such a movement may be termed a bind-release movement. Thus, the CMM surface may be configured not to bind a cell indefinitely. Binding and releasing the cell may temporarily halt the cell along its travel. Such a modified motion in the form of a modified speed may be detectable by the at least one detector. The cell may not necessarily bind and release at the same point of the CMM surface. The CMM surface may be configured to let a cell bind to the CMM surface, roll a distance along the CMM surface, and then release from the CMM surface in a bind-roll-release movement. The cell may e.g. roll along a surface in a direction different from the direction of the flow of liquid. Thus, the direction or path of a cell belonging to a category associated with the CMM surface may be different from the path of a cell belonging to a category not associated with the CMM surface. Such a modified motion in the form of a modified direction or path may be detectable by the at least one detector.

In some embodiments the flow channel may comprise more than one zone. The at least one zone of the flow channel may comprise at least a first zone and a second zone, wherein a composition of the molecules coating the at least one CMM surface of the first zone differs from a composition of the molecules coating the at least one CMM surface of the second zone. Within the flow channel the second zone may be arranged downstream from the first zone.

Several zones with different CMM surfaces may enable modified movements of the cells belonging to multiple cell categories. The combination of information regarding the modified movements of cells in different zones may also enable complex analysis the cells. For instance, a biological cell may be subject to modified movements in several zones and different biological cells may be distinguished from each other based on in which combination of zones the biological cells are subject to modified movements.

The at least one detector is configured to detect information representing the biological cells passing the at least one zone. The at least one detector may be configured to detect one modified movement of a cell in a zone, e.g. one bind and release event, a halt of motion, a change of speed, a change of direction, or a change of path, and thereby identify the cell as belonging to the cell category associated with the zone. Alternatively, the detector may be configured to detect a threshold number of modified movements of a cell in a zone, e.g. the cell displaying at least 2, at least 5, or at least 10 bind and release events within the zone, and thereby identify the cell as belonging to the cell category associated with the zone. The at least one detector may be configured to detect modified movements of two or more cells simultaneously, as they move across a CMM surface of the flow channel.

The at least one detector may further be configured to detect information on shape and size of the biological cells.

The at least one detector may be configured to acquire images of the biological cells in the flow channel. The at least one detector may be configured to acquire an image series representing a time-sequence of images of the biological cell. From the images, the processor may process information regarding the biological cells.

The image acquired by the detector may be a 2D array of intensities, such as a hologram. The hologram may then include information of the area detected by the detector. The hologram may be used to reconstruct an image for the user to see. However, the processor may use the information in the hologram without reconstructing an image,

It should be understood that the detector may comprise multiple image sensors. The multiple image sensors may image a larger area than a single image sensor could. Each image sensor may have its own light source. Alternatively, two or more image sensors may share a light source. The field of view of an image sensor may or may not overlap with the field of view of another image sensor.

The processor is configured to receive the information detected by the at least one detector. Thus, the processor may be configured to analyze a time-sequence of information of the biological cells and detect the cell as exhibiting the at least one modified movement based on the time-sequence of information.

The processor divides the at least one zone of the flow channel into segments. It should be understood that it is not a physical division into segments, but the processor logically defines the segments of each of the zones for analysis of the tracks of the biological cells. Thus, the division of the zones into segments will not affect the biological cells passing through the zone but is rather only a computational way of making the analysis.

The processor identifies candidate biological cells. The candidate biological cells are identified from the information detected by the at least one detector. The candidate biological cells may be biological cells belonging to the cell category of the zone. The candidate biological cells may be a biological cell not belonging to the cell category of the zone. The candidate biological cell may however also be non-cell. The non-cell could be a debris in the flow of liquid, a dead or injured cell, an artefact in the CMM surface of the zone or an imaging artefact interpreted by the detector as a cell.

The processor of the device is configured to process information acquired by the detector such that it may decide whether a candidate biological cell is actually a biological cell of interest or not.

Each candidate biological cell may be represented by a track that defines a path of a movement of the candidate biological cell through the at least one zone of the flow channel. The track may represent the position of a detected candidate biological cell in the flow channel over a time-sequence during which the candidate biological cell passes the zone. Thus, the movements of the each of the candidate biological cell passing the zone is tracked as well as the time the candidate biological cell spends in the zone. The detector acquires information on movement in space and time of the candidate biological cell and may provide such information to the processor.

The track may represent a set of data points, where each data point represents a timepoint and the position of the candidate biological cell at least in two dimension but even in three dimensions. Thus, each track comprises information of x, optionally y, and optionally z, and t, where x, y and z are spatial coordinates and t is the timepoint.

For each of the tracks, a set of transit times are determined. Thus, each candidate biological cell has a set of transit times defining its passing through the at least one zone. Each transit time represents a transit time of the candidate biological cell through a segment. Thus, for each segment the transit time is detected such that the set of transit times represents the time for the biological cell to pass the zone.

For each candidate biological cell, the set of transit times are processed.

The processing may be used to characterize the candidate biological cell by determining if the candidate biological cell is a biological cell belonging to an associated biological cell category of each respective zone.

If the candidate biological cell belongs to an associated biological cell category of the zone, there is a high probability that its movement is modified by the CMM surface. This may be detected by comparing for instance how long a candidate biological cell spends in the zone. Thus, the set of transit times for a cell associated with the biological cell category of the zone may include transit times through one or more segments that are longer than transit times of a cell not belonging to the biological cell category associated with the zone.

The use of segments in the zone of the flow channel ensures that a plurality of transit times through respective segments of the zone are determined. This enables the processing of the set of transit times to identify variations in the transit times within the set, which is very useful for identifying the candidate biological cells that actually are biological cells belonging to the cell category associated with the zone. For instance, if only a single overall transit time through the entire zone would be used, the processor would have difficulty to distinguish between a biological cell belonging to the biological cell category associated with the zone from other cells or non-cells that may for some reason be passing through the zone at a general lower speed than expected.

In other words, the processing of the transit times may reveal if the candidate biological cell has interacted with the CMM surface or not. It should be realized that the set of transit times may be processed in many different manners in order to determine the probability that the candidate biological cell is a biological cell belonging to an associated biological cell category of each respective zone.

Each zone comprises a surface coated with molecules having an affinity and specificity to a cell category associated with the zone. It should be realized that the cell category may thus be purely defined as a group of cells that will be subject to modified movements. This group of cells may or may not have a correspondence with a conventionally used manner of defining cell categories. It should further be realized that a determination of a category of a biological cell into conventionally used categories may be based on movement of the cell through a plurality of zones having surfaces coated with different molecules and a combination of zones among the plurality of zones in which the cell is subject to specific modified movements.

During processing, the order of the transit times through different segments may be useful. For instance, the order may reveal correlations between events where several candidate biological cells have a similar transit time through a specific segment of the zone. The order of the transit times may also reveal if there is for instance a sequence of 5 long transit times in consecutive segments indicating that the candidate biological cell has a lower speed while passing through a relatively long distance of the zone or if there are 5 separate long transit times through separate, non-consecutive segments.

However, it should also be understood that the transit times may be processed independently of their order such that only the lengths of the transit times within a set are relevant for the processing.

According to one embodiment the processing of the set of transit times may comprise determining at least one parameter representing a sequence of transit times within the set of transit times, and characterizing the candidate biological cell based on the at least one parameter.

Thus, the set of transit times may be analytically processed.

The transit times of each of the candidate biological cells passing the zone and tracked by the processor may be processed by investigating the distribution of the segment transit times. From the distribution, parameters such as mean transit time, standard deviation of all transit times of candidate biological cell, skewness of the distribution of all segment transit times, may be determined.

The parameters from each of the candidate biological cells may be compared to each other. For instance, the skewness of the transit times may be plotted against the standard deviation of the transit times. This may result in clusters, such that the candidate biological cells belonging to the biological cell category associated with the zone may have similar skewness and standard deviation of the transit times. Further, the candidate biological cells not belonging to the biological cell category associated with the zone may have a similar skewness and standard deviation of the transit times. The skewness and the standard deviation of the candidate biological cells belonging to the biological cell category associated with the zone may differ from the skewness and standard deviation of the candidate biological cells not belonging to the biological cell category associated with the zone.

It should be understood that skewness and standard deviation is only two examples of descriptive parameters which may be used to classify the candidate biological cells based on their transit time over the CMM surface.

According to one embodiment the determining at least one parameter representing a sequence of transit times may further comprise defining at least one subset of the at least one parameter and characterizing the candidate biological cell based on the at least one subset.
Thus, the parameters may be compared and divided into subsets. The subset of the parameters may indicate candidate biological cells being similar and thus for instance belonging to the associated cell category of the zone.

According to one embodiment the processing of the set of transit times may be made by use of a machine learning model.

The model may be a supervised machine learning model.

By using a machine learning model, the classification may be made with a high throughput and a large number of candidate biological cells may be characterized. The machine learning model may be trained to correctly classify candidate biological cells finding patterns for characterizing candidate biological cells based on the set of transit times which may not be evident through analytical analysis of the set of transit times.

According to one embodiment the characterization of the candidate biological cells may comprise a classification of the candidate biological cells.

The classification may for instance classify the candidate biological cells into pre-known groups. For instance, the classification may determine if the candidate biological cell with a high probability has a certain marker on the cell surface, by analyzing its transfer through the zone.

According to one embodiment the processor may be configured to divide each zone into at least 100 segments.

A large number of segments allows for a more precise segment time and even smaller differences can be measure compared to longer and fewer segments. However, a large number of segments also gives a large amount of data. Thus, the data processing may be more time consuming with a large amount of segments.

As an example, a zone of 10 mm may be divided into approximately 400 segments. Thus, each segment represents approximately 25 µm of the zone. This gives a high resolution for the classification of the candidate biological cells.

The number of segments may thus be 10, 100, 200, 300, 400, 500, 600, 700, 800, 900 or 1000 for each zone.

It should be realized that at least in some embodiments, the processor may be configured to divide each zone into only 2 segments. This may still provide a possibility of processing the set of transit times for correctly characterizing the candidate biological cell.

According to one embodiment the processor may be configured to divide the flow channel into segments outside of the at least one zone.

By this, the segments outside the at least one zone may give reference information of each candidate biological cell, such that each candidate biological cell has a control value for transit times.

For instance, if a candidate biological cell passes the segments of the zone with a transit time being shorter or significantly shorter than the transit times for passing the segments outside of the zone, it may indicate that the movement of the candidate biological cell is modified by the CMM surface and thus that he candidate biological cell belongs to the biological cell category associated with the zone. On the other hand, if there are no or very small or random differences between the transit times of a candidate biological cell when passing the segments outside of the zone and inside of the zone it may indicate that the candidate biological cell does not belong to the candidate biological cell category associated with the zone.

For this, the at least one detector is further configured to detect information representing the biological cells, carried by the flow of liquid, passing the segments outside of the at least one zone.

According to one embodiment the flow channel may comprise a plurality of zones, each having an affinity and specificity to a separate cell category.

This allows for classification of more than one cell category in one sample. For instance, a first zone may have an affinity for CD4 positive cells, while a second zone may have an affinity for CD8 positive cells. Then, CD4 positive cells may have longer transit times while passing through the first zone and CD8 positive cells may have longer transit times while passing through the second zone.

Each zone may be divided into segments. The number of segments may be the same between the zones. However, the number of segments may also differ between the zones.

According to one embodiment the flow channel may comprise a plurality of zones, each having an affinity and specificity to a different cell category.

An advantage of this is that the result may be more accurate, as the candidate biological cells passes several zones having the same CMM surface, and thus the probability of modification of movements of all biological cells belonging to the cell category associated with the zone increases.

According to one embodiment the processor may be configured to divide each zone into segments having a fixed and equal length.

Thus, the length of all segments of one zone has the same length. In other words, the zone is equally divided into segments.

This allows for a comparison of transit times between the segments within the zone.

However, the length of segments may also differ between the zones.

According to one embodiment, the processor may be configured to divide each zone into segments having a fixed and equal transit time for candidate biological cells whose movement is not modified by the CMM surface.

Thus, the processor may divide the zone into segments based on transit times, which for simplicity may be called time segments. The time segments represent a division of the zone where the time to pass the segment is defined by the processor before or after the passing of the cells. As already discussed, this is not a physical division into segments, but the processor logically defines the segments of each of the zones for analysis of the tracks of the biological cells. For the time segments, the length of the segment is dependent on the (actual or expected) time it takes for cells not interacting with the CMM surface to pass the segment. Thus, the actual length of the segments may vary as the speed of cells not interacting with the CMM surface may differ through the at least one zone. A cell not interacting with the CMM surface may for instance pass the zone in an equal speed through all time segments. The time segments may then be set to this transit time and any candidate biological cell diverting from this may be identified. It should be understood that the time segments based on the transit time for candidate biological cells whose movement is not modified by the CMM surface may be the expected transit time during nominal behavior of a cell not interacting with the CMM surface.

Dividing each zone into segments having fixed and equal transit times may for instance also allow for detection of any abnormal behavior caused by for instance an artefact in the zone. If a transit time of one or a few segments in a certain part of the flow channel generally differs a lot from the other transit times for all or many candidate biological cells, it may reveal that the passage through that part of the zone may be affected by a general defect and may not be due to a specific modified movement by the CMM surface. This may further show up as prolonged transit times of all candidate biological cells, since artefacts may physically affect the speed of transition for all candidate biological cells passing the affected segment.

Thus different parts of the flow channel may have time segments with different physical length but equal transit time, i.e. equal time segments over the zone may be connected to a different length of the time segment.

It should be understood that the segments which the processor is dividing each zone into, according to the first aspect, could be the segments having a fixed and equal length and/or the segments having a fixed and equal transit time for candidate biological cells whose movement is not modified by the CMM surface of the zone.

According to an embodiment, the processor may be configured to apply a transformation on the information detected by the detector and/or on identified tracks for mapping tracks of candidate biological cells whose movement is not modified by the CMM surface of the zone onto straight paths through a virtual flow channel.

The transformation thus renders a virtual flow channel in which the tracks get a straight path. Thus, complicated movement patterns may be transformed into a more simple pattern allowing for a more simple analysis. By the transformation, both the length and the shape of the segments may be transformed into the virtual flow channel and get a more simple shape.

Candidate biological cells whose movement is not modified by the CMM surface have a high probability of being candidate biological cells not belonging to an associated biological cell category of the zone.

An advantage of this is that candidate biological cells not interacting with the CMM surface, may be transformed into having a constant velocity and any expected variations in motion, e,g, in a channel with a complex shape or which contains obstacles, may be removed from the information in the track by the transformation.

The transit time of candidate biological cells belonging to the cell category associated with the at least one zone may differ from the transit time of candidate biological cells not belonging to an associated biological cell category of the zone passing through the time segments.

According to an embodiment, the processor may be configured to normalize the transit times for each candidate biological cell and for each segment. The normalization may be done by dividing the transit time for each candidate biological cell by the expected behavior of cells not belonging to the category of the zone. This normalization would allow for a simple way of analyzing flow channels of complex shapes, or for channels having obstacles. It may further allow for a compensation for candidate biological cells being stuck during the flow through the at least one zone.

It should be understood that velocities of candidate biological cells passing through the at least one zone can first be normalized, e.g., by dividing the velocity with the expected velocity of cells not belonging to the category of the zone, before a segmentation of the at least one zone is made and the transit times are determined.

According to an embodiment, the processor may be configured to perform a weighted analysis of the transit times of each segment, wherein the segments may be then given different weights in the further analysis. Thus, the transit times of each segment may be analyzed and based on that, the processor may decide if any segment is of higher or lower importance. For instance, if the zone is covered by pillars or other obstacles, it may be of more interest to analyze the segments covering such pillars. Likewise, knowing that a part of a zone, for instance before or after a pillar, may give rise to abnormal movements, may make it of less interest to analyze and therefore, that segment may be given less weight in the analysis.

An advantage of this is that the characterization may be based on the segments comprising the most relevant information, such that information on modified movements is not hidden among movements caused by other sources than the CMM surface.

According to one embodiment, the processor may be configured to divide each zone into individual segments for each of the candidate biological cells. Thus, candidate biological cells taking different paths through the at least one zone may have passed a different number of segments and may have different number of transit times.

The basis for the division into segments may be divided based on the shape of the flow channel.

Segments may be removed if the analysis reveal that any segment comprises any general problem, such as a debris.

An advantage of this is that candidate biological cells taking completely different paths through the zone may still be comparable, when taking into account the different paths.

According to one embodiment the processor may be configured to divide each zone into segments, each having a length of approximately 25 µm, such as between 20 - 30 µm.

The length of the segments may vary between devices. The length of the segments may be dependent on the at least one detector. The length of the segments may vary depending on the kind of candidate biological cells to be characterized. Fast moving cells may need longer segments than slow moving cells. The length of the the segments may vary depending on the flow speed.

Thanks to the fact that the segments are defined only by the processor, the number of segments and the length of them may be varied to fit the specific need for the classification and the type of biological cells.

The length of the segments may thus be 5, 10, 20, 25, 30, 35, 40, 45, 50, 100, or 1000 µm.

The shape of the segments or the boundaries between segments may differ between zones, if the device comprises multiple zones.

According to one embodiment the detector may be an image sensor.

Thus, the information detected by the at least one detector may be imaging data. Thus, the transit times may be calculated by measuring in the image data how far the candidate biological cell has moved during a specific time.

According to one embodiment the device may further comprise:
a light source configured to illuminate a biological cell in the flow channel such that an interference pattern is formed by interference between light being scattered by the illuminated biological cell and non-scattered light from the light source; and
wherein the image sensor is configured to detect an image series that represents a time-sequence of interference patterns of the illuminated biological cell.

Such a detector may enable a high cell throughput. A detector operating according to the principles of digital holographic imaging may have a large field of view since a lens may not be needed between the image sensor and the flow channel. Further, a detector operating according to the principles of digital holographic imaging may have a large depth of focus. Consequently, the detector may monitor a large area or a large volume, of the flow channel, for cells exhibiting modified movements. Monitoring a large area or volume simultaneously makes it possible to maintain a high flow rate and a high cell throughput.

The non-scattered light from the light source may be passed along a common optical path with the light being scattered by the cells. Thus, the interference pattern may be formed between scattered and non-scattered light at the image sensor in a so-called in-line holography set-up. Thus, the scattered and non-scattered light may share the same light path. However, according to an alternative, the non-scattered light may be passed along a separate reference light path, which is combined with the light having been scattered by the cells before reaching the image sensor. In such case, the light scattered by the cells may be either forward or backward scattered light.

It should be realized that the interference pattern may be used for reconstructing images of the flow channel. Thus, such reconstructed images may be provided to the processor as information representing the biological cells carried by the flow of liquid. However, the processor may be able to identify tracks in the time-sequence of interference patterns without the interference patterns being converted to reconstructed images.

When the detector comprises a light source, configured to illuminate an object in the flow channel, and an image sensor configured to detect an image series representing a time-sequence of interference patterns of the illuminated object, the light source may be configured to emit at least partially coherent light. Coherent light may be advantageous as it may improve the interference visibility. A coherent light source may be a laser. However, it should be understood that also partially coherent light may provide an interference pattern with sufficient visibility. A partially coherent light source may e.g. be a light emitting diode which emits light directly onto the flow channel or through a pinhole onto the flow channel. A coherent light source may provide better interference visibility but be more expensive while a partially coherent light source may provide a worse interference visibility but be less expensive.

As an alternative to a detector operating according to the principles of digital holographic imaging, the detector may use imaging with a lens to detect cells exhibiting modified movements. Thus, the detector may detect an image series representing a time-sequence of a cell in the flow channel instead of a time-sequence of interference patterns of the cell.

However, it should be realized that information relating to tracks may be acquired by detecting changes in electrical properties in the flow channel associated with movements of objects therethrough. Thus, the at least one detector may instead be an electrical sensor configured to detect an electrical signal that is affected by biological cells passing through the zone, such that the electrical signal may be used for sequentially determining a location of the candidate biological cells in the zone of the flow channel. The electrical sensor may be an array of electrical sensors.

According to a second aspect, there is provided a method for characterizing biological cells, the method comprising:
receiving information detected by at least one detector representing biological cells, carried by a flow of liquid, to be characterized passing at least one zone in a flow channel, wherein
   each zone of the flow channel is associated with a cell category,
   each zone of the flow channel comprises at least one surface coated with molecules having an affinity to the cell category associated with the zone, the at least one surface of the zone being configured to modify, by the molecules coating the at least one surface, a movement of a biological cell belonging to the associated cell category of the zone as the biological cell carried by the flow of liquid passes the zone, whereby the at least one surface forms at least one biological cell movement modifying (CMM) surface, and whereby the modified movement of the biological cell caused by the CMM surface forms a specific modified movement,
processing the received information , wherein said processing of the received information comprises:
   dividing each zone of the flow channel into segments;
   identifying candidate biological cells in the flow channel;
   identifying tracks from each of the candidate biological cells;
   for each track of the candidate biological cells, determining a set of transit times, wherein each transit time in the set represents a transit time of the candidate biological cell through a segment;
   for each candidate biological cell, processing the set of transit times for characterizing the candidate biological cell.

This aspect may generally present the same or corresponding advantages as the former aspect.

The method allows for characterization of biological cells. The method is based on that the movement of the biological cells are modified when passing through the zone with a CMM surface. The zone is divided into segments by the processor and the time it takes for the biological cell to pass each segment is determined as transit times.

The transit times are processed to characterize the biological cells.

The processing of the transit times may be made by comparing the transit times between several biological cells passing through the segments of the zone. Thus, the transit times are used to determine what cells are interacting with the CMM surface and which are not interacting with the CMM surface. Interaction with the CMM surface generally gives longer transit times and thus the length of the transit times may be used to classify the biological cells.

According to a third aspect, there is provided a computer program product comprising computer-readable instructions such that when executed on a processor, the computer program product will cause the processor to perform the method according to above.

This aspect may generally present the same or corresponding advantages as the former aspects.

The computer program product may implement the method in a processing unit, which may be a dedicated processing unit for performing the method or may be a general-purpose processing unit which may be able to perform the method based on the computer program product.

The computer program product may comprise a computer-readable medium on which the computer-readable instructions are stored. The computer program product may thus be provided as a non-transient computer program product stored on any tangible medium. Alternatively, the computer program product may be provided as a signal carrying the computer-readable instructions for allowing the computer program product to be loaded into a memory accessible to the processing unit.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a device for analysis of biological cells.
Fig. 1b is a device for analysis of biological cells.
Fig. 2a is a device for analysis of biological cells.
Fig. 2b is a device for analysis of biological cells.
Fig. 3 is a device for analysis of biological cells.
Fig. 4a is a device for analysis of biological cells.
Fig. 4b is a device for analysis of biological cells.
Fig. 4c is a schematic of biological cells.
Fig. 4d is a device for analysis of biological cells.
Fig. 4e is a device for analysis of biological cells.
Fig. 5 is a flow chart of a method for analyzing biological cells.

### Detailed description

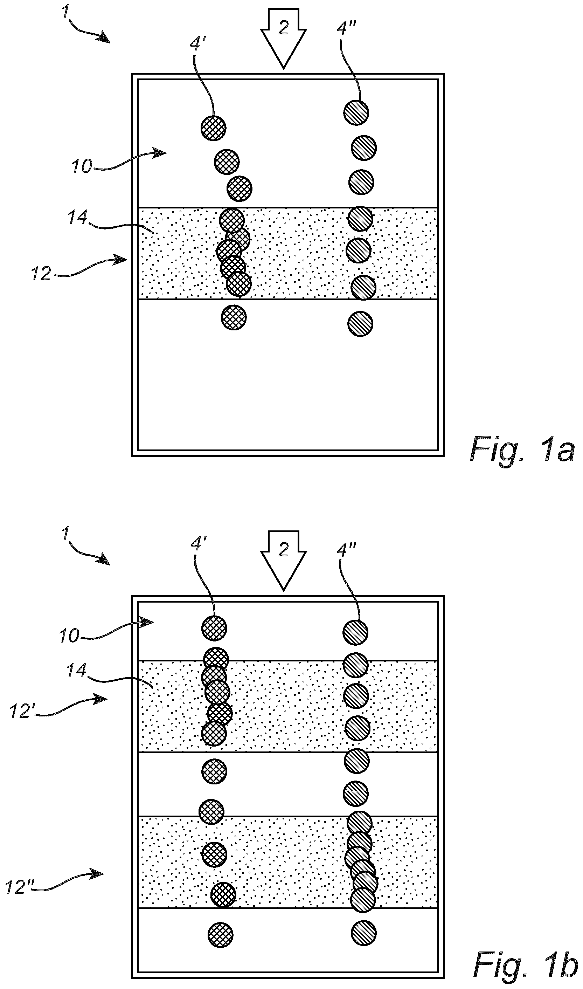

Figs 1a and 1b illustrate a flow channel 10 of a device 1 for analyzing biological cells 4. The flow channel 10 is configured to pass a flow 2 of liquid carrying the cells 4 to be analyzed. The flow channel 10 may be a microfluidics channel. The flow channel 10 may be defined by conventional lithography and sealed with a transparent cover slide, e.g. glass, PDMS, or polycarbonate. The top part of the flow channel 10 may be covered with a cover slide, e.g. a glass slide, bonded to the PDMS.

In Fig. 1a, the illustrated flow channel 10 comprises one zone 12. The flow 2 of liquid and the biological cells 4 passes the zone 12. The bottom part of the zone 12 are covered with molecules such that they form a cell movement modifying (CMM) surfaces 14. The CMM surfaces 14 comprise molecules having an affinity to a cell category.

In Fig. 1a, in the illustrated flow channel 10, the CMM surfaces 14 of the zone 12 are coated by molecules having an affinity to biological cells 4' of a first category. Thus, the zone 12 may be considered to be associated with the first category.

In Fig. 1b, in the illustrated flow channel 10, the CMM surfaces 14 of the first zone 12' are coated by molecules having an affinity to cells 4' of a first category. Thus, the first zone 12' may be considered to be associated with the first category. In the illustrated flow channel 10, the CMM surfaces 14 of the second zone 12" are coated by molecules having an affinity to cells 4" of a second category. Thus, the second zone 12" may be considered to be associated with the second category.

The molecules of the CMM surfaces 14 may be e.g. antibodies, aptamers, antibody fragments, peptides, molecularly imprinted polymers, receptors or lectins. For example, the molecules of the CMM surface 14 of the first zone 12' may be anti-CD4 antibodies and the molecules of the CMM surface 14 of the second zone 12" may be anti-CD8 antibodies. Thus, a first cell category may be defined as biological cells 4' having a cell membrane comprising CD4 antigens ("CD4 positive cells") and a second cell category may be defined as biological cells 4" having a cell membrane comprising CD8 antigens ("CD8 positive cells").

It should be realized that CMM surfaces may be provided on side walls or on the top surface of the flow channel instead or in addition to on the bottom part of the flow channel.

As illustrated in Fig. 1a and 1b, the molecules of the CMM surfaces 14 may modify the speed of the movement of the cells 4. The modified movements may be expressed as cells 4 have a reduced speed if they interact with the CMM surfaces 14 of a zone 12.

In Fig. 1a, a biological cell 4' of the first category have a slower speed in the zone 12 compared to the speed of the biological cell 4" of the second category.

In Fig. 1b, a biological cell 4' of the first category has a slower speed in the first zone 12' than in the second zone 12". Analogously, a biological cell 4" of the second category has a slower speed in the second zone 12" than in the first zone 12'.

However, one candidate biological cell 4', 4" may belong to more than one cell category. Thus, one candidate biological cell 4', 4" may belong to both the associated cell category with the first zone 12' and the second zone 12".

The molecules may bind and release a cell 4 belonging to the associated cell category of the zone 12. For example, an anti-CD4 antibody may bind to a cell 4 having a cell membrane comprising CD4 antigens, and subsequently release the cell. By this, the speed of a biological cell 4' of the first category moving through the first zone 12' may be reduced such that it is possible to identify the cell as belonging to the first category. The movements of a biological cell 4" of the second category moving through the second zone 12" may change analogously.

When a biological cell belongs to a category associated with a zone the biological cell may, in that zone, exhibit e.g. a bind and release event, a halt of motion, a change of speed, a change of direction, or a change of path.

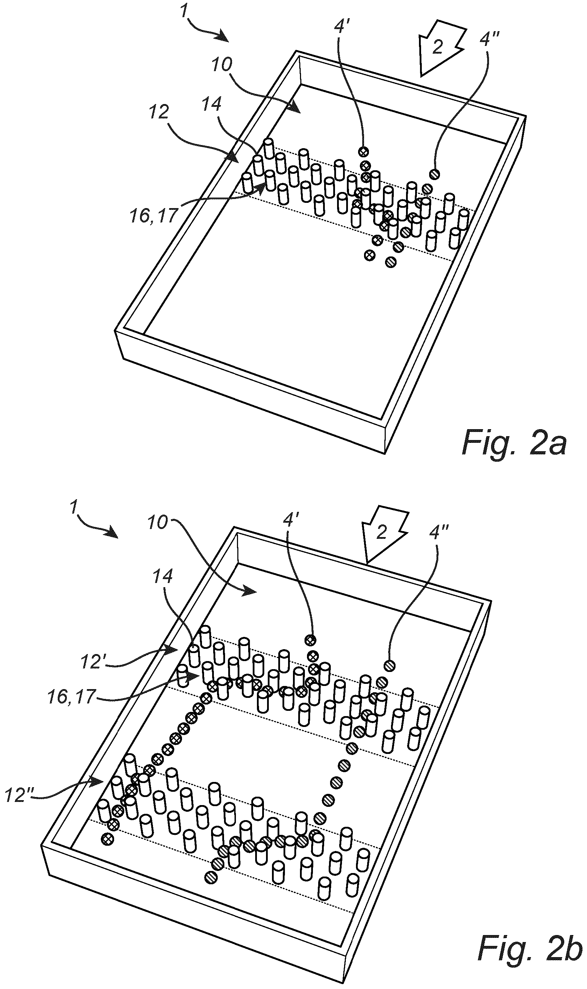

Figs 2a and 2b illustrate a flow channel 10 of a device 1 for analyzing biological cells 4. The device 1 have many similarities to the device 1 described in Figs. 1a and 1b, thus only differences between the figures will be disclosed.

In Fig. 2a, the illustrated flow channel 10 comprises one zone 12. The flow 2 of liquid and the biological cells 4 passes the zone 12. The zone 12 comprises obstacles 16, in the figure obstacles 16 in the form of pillars 17.

In Fig. 2b, the illustrated flow channel 10 comprises two zones 12. The flow 2 of liquid and the biological cells 4 first passes a first zone 12' and then a second zone 12". Each zone 12 comprises obstacles 16, in the figure obstacles 16 in the form of pillars 17.

Except for the number of zones, the device 1 may be configured in the same way regardless of the device 1 having one or several zones 12. It should be understood that the device may have more than two zones.

In the illustrated device 1, the side walls of the pillars 17 form lateral surfaces that obstruct the path of the biological cells 4. Thus, the biological cells 4 repeatedly hit the side walls of the pillars 17 during their travel through the flow channel 10. Further, the side walls of the pillars 17 form cell movement modifying (CMM) surfaces 14. The CMM surfaces 14 comprise molecules having an affinity to a cell category.

In Fig. 2a, in the illustrated flow channel 10, the CMM surfaces 14 of the zone 12 are coated by molecules having an affinity to biological cells 4' of a first category. Thus, the zone 12 may be considered to be associated with the first category.

In Fig. 2b, in the illustrated flow channel 10, the CMM surfaces 14 of the first zone 12' are coated by molecules having an affinity to cells 4' of a first category. Thus, the first zone 12' may be considered to be associated with the first category. In the illustrated flow channel 10, the CMM surfaces 14 of the second zone 12" are coated by molecules having an affinity to cells 4" of a second category. Thus, the second zone 12" may be considered to be associated with the second category.

As illustrated in Fig. 2a and 2b, the molecules of the CMM surfaces 14 may modify the movement of the cells 4. The modified movements may be expressed as cells 4 taking a characteristic type of path if they interact with the CMM surfaces 14 of a zone 12.

In Fig. 2a, a biological cell 4' of the first category takes a more undulating path in the zone 12 compared to the path of the biological cell 4" of the second category.

In Fig. 2b, a biological cell 4' of the first category takes a more undulating path in the first zone 12' than in the second zone 12". Analogously, a biological cell 4" of the second category takes a more undulating path in the second zone 12" than in the first zone 12'.

The molecules may bind and release a cell 4 belonging to the associated cell category of the zone 12. For example, an anti-CD4 antibody may bind to a cell 4 having a cell membrane comprising CD4 antigens, and subsequently release the cell. Thus, the movements of a biological cell 4' of the first category moving through the first zone 12' may change in a characteristic way that makes it possible to identify the cell as belonging to the first category. The movements of a biological cell 4" of the second category moving through the second zone 12" may change analogously. When a biological cell belongs to a category associated with a zone the biological cell may, in that zone, exhibit e.g. a bind and release event, a halt of motion, a change of speed, a change of direction, or a change of path. Fig. 2a and 2b illustrates a cell 4' of the first category which, in the first zone 12', follows the curvature of the pillars 17 to an extent that indicates that the cell does not merely follow the flow 2 of liquid.

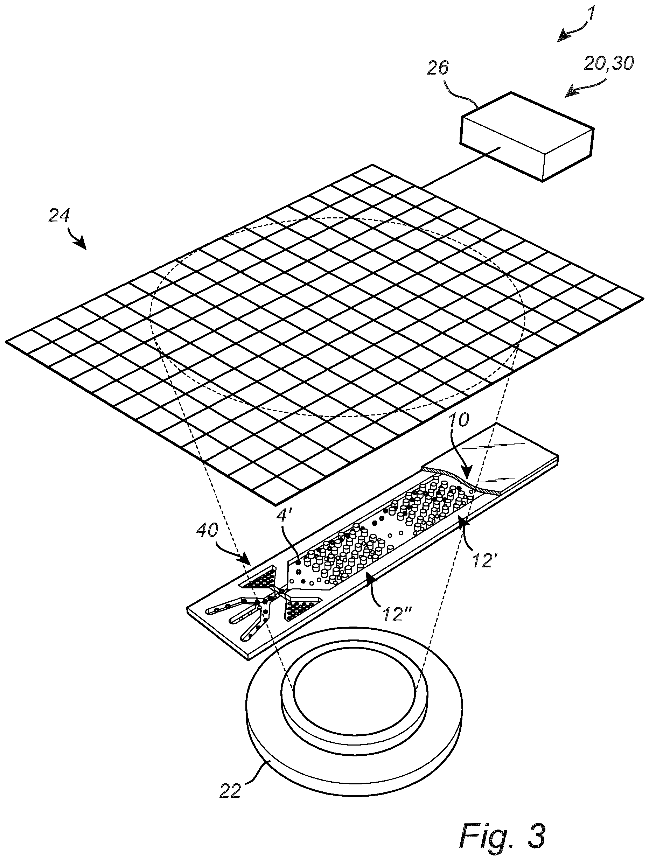

Fig. 3 illustrates a device 1 comprising a detector 20, being an image sensor 24 and a light source 22. The illustrated light source 22 illuminates the zones 12 of the flow channel 10. The flow channel 10 of the device 1 in Fig. 3 is the flow channel 10 depicted in Fig. 2b.

The illustrated light source 22 is configured to illuminate the cells 4 as they pass through the flow channel 10. The light source 22 may herein be a coherent light source 22, e.g. a laser, or a partially coherent light source 22, e.g. a light emitting diode or a light emitting diode with a pin hole or aperture. As the cells 4 are illuminated an interference pattern is formed on the image sensor 24. The image sensor 24 may comprise a plurality of photo-sensitive elements configured to detect incident light. The image sensor 24 may herein be a CCD or CMOS camera. The image sensor 24 may acquire a time-sequence of image frames of the changing interference pattern as cells 4 pass the image sensor 24. In the figure the light source 22 and the image sensor 24 are arranged on opposite sides of the flow channel 10. The flow channel 10 is herein at least partially transparent such that the light may travel through the flow channel 10.

The detector 20 is configured to detect information representing the biological cells 4, carried by the flow of liquid 2, passing the at least one zone 12. The at least one detector 20 may be configured to detect information representing two or more biological cells 4 simultaneously, as they move across a CMM surface of the flow channel.

The at least one detector 20 may be configured to detect information of at least one property of the biological cells 4. The property may be the size, shape or solidity of the biological cells 4.

The at least one detector 20 may be an optical sensor or the device 1 may further comprise an optical sensor. The at least one detector 20 may be an electrical sensor or the device 1 may further comprise an electrical sensor.

The device 1 further comprises a processor 26 which is configured to receive the information detected by the at least one detector 20.

The processor 26 may e.g. perform a holographic reconstruction of two interference patterns detected by the detector 20 in the time-sequence of interference patterns. Said two reconstructions may depict e.g. a biological cell 4 in contact with a CMM surface 14.

The processor 26 may not necessarily perform a full holographic reconstruction of two interference patterns in the time-sequence of interference patterns. The cell 4 may be detected as exhibiting a modified movement based on two, or more, interference patterns in the time-sequence of interference patterns or partial holographic reconstructions of the two, or more, interference patterns.

The device 1 may be configured to detect modified movements of biological cells carried by the flow of liquid in a manner described in WO2022/241138 A1, which is hereby incorporated by reference.

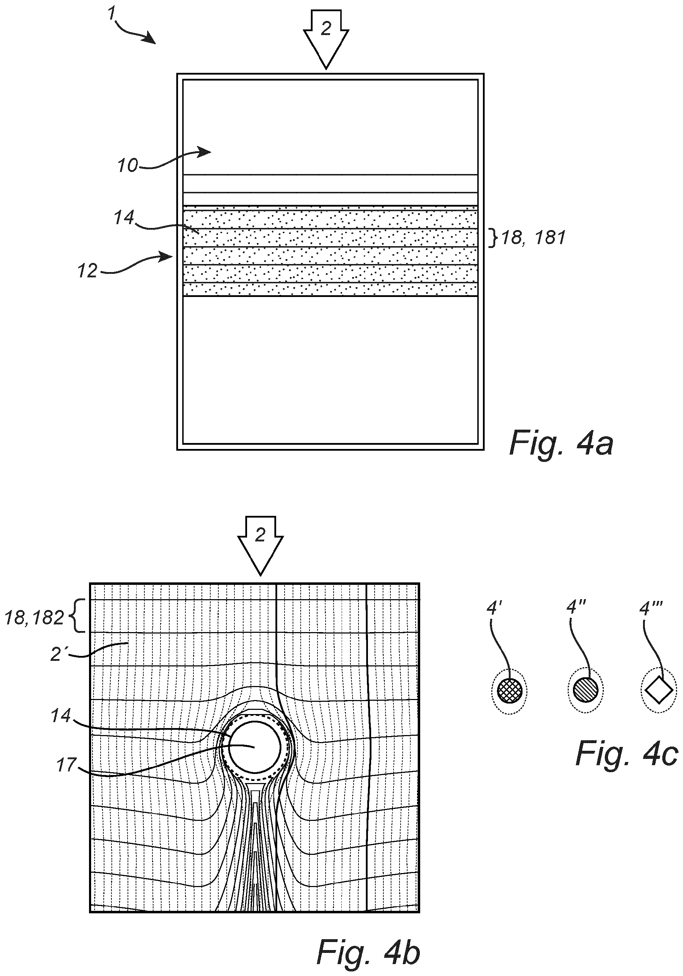

The processor 26 is configured to process the information received from the at least one detector 20. The processing of the information will now be discussed in relation to Fig. 4a to c.

The processor 26 may be a central processing unit (CPU), which may execute the instructions of one or more computer programs in order to process the information received from the at least one detector 20.

The processing unit 120 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality for processing the information received from the at least one detector.

The processing of the information comprises dividing each zone 12 of the flow channel 10 into segments 18, as illustrated in Fig 4a. The processor 26 logically defines the segments 18 of each of the zones 12 for analysis of the tracks 41', 41", 41‴ of the biological cells 4', 4", 4‴. The segmentation of the at least one zone 12 is a computational process and it will not affect the candidate biological cells 4', 4", 4‴ passing the zone 12.

The number of segments 18 may be 10, 100, 200, 300, 400, 500, 600, 700, 800, 900 or 1000 for each zone 12.

Each zone 12 may be divided into segments 18 having a fixed and equal length 181. The length of the segments 18,181 may be 5, 10, 20, 25, 30, 35, 40, 45, 50, 100 or 1000 µm.

For instance, a zone 12 of 10 mm may be divided into 400 segments 18, 181. Thus, each segment 18, 181 represents 25 µm of the zone 12.

The processor 26 may further be configured to divide the flow channel 10 into segments 18 outside of the zone 12 as well.

Alternatively, as is illustrated in Fig. 4b, having a channel with a complex shape or which contains obstacles 16 e.g. pillars 17, with e.g. a CMM surface 14 on the sidewalls of the obstacles. Each zone 12 may, by the processor 26, be divided into segments having a fixed and equal transit time 18, 182 for candidate biological cells 4, 4', 4", 4‴ whose movement is not modified by the CMM surface 14. Those segments 182 may for simplicity be called time segments 182.Thus, candidate biological cells having a high probability of not belonging to an associated biological cell category of the zone 12, 12', 12' may have a fixed an equal transit time through the time segments 182 The transit time of a biological cell 4, 4', 4", 4‴ belonging to an associated biological cell category of the zone 12, 12', 12" has a high probability of having a transit time differing from the fixed and equal transit time.

As is visualized in Fig. 4b, the physical length of the time segments 182 are unequal through the at least one zone. This is caused by the candidate biological cells 4, 4', 4", 4‴ having different paths and velocities through the at least one zone 12, 12', 12" as some candidate biological cells 4, 4', 4", 4‴ pass close by the pillar 17 and some passes further away from the pillar 17. This also means that the number of time segments over the at least one zone may differ, as the transit time for candidate biological cells 4, 4', 4", 4‴ whose movement is not modified by the CMM surface 14 over the time segments 182 are equal, but the total time to pass the at least one zone 12, 12', 12", 12‴ differs depending on where the candidate biological cell moves 4, 4', 4", 4'". This is visualized in Fig 4b by two thicker paths.

For instance, a zone 12 having a passing time of approximately 1 minute for candidate biological cells 4, 4', 4", 4‴ not belonging to an associated biological cell category of the zone 12, 12', 12" may be divided into time segments 182 having a length of 10 seconds each. This could possibly give five, six, seven or any other number of time segments 182 of 10 seconds each.

Dividing the zone 12, 12', 12" into time segments may be used to correct for abnormalities in the flow, such that candidate biological cells 4, 4', 4", 4‴ may be affected by debris or any other anomality in the at least one zone 12, 12', 12", changing the transit times of the candidate biological cells 4, 4', 4", 4‴ passing the spot of the anomality. Thus, dividing into time segments 182 may reveal that time segments 182 covers different length of the at least one zone 12, 12', 12", in other words that it may take different time for the candidate biological cells 4, 4', 4", 4‴ to pass a certain distance of the at least one zone 12, 12', 12".

The processor 26 may be configured to apply a transformation on the information detected by the detector and/or on identified tracks 41', 41", 41‴ for mapping tracks of candidate biological cells 4, 4', 4", 4‴ whose movement is not modified by the CMM surface 14, 14', 14" of the zone 12, 12', 12" onto straight paths of a virtual flow channel.

During movement through the at least one zone 12, 12', 12", the candidate biological cells 4, 4', 4", 4'", may have undulating paths due to several reasons, as discussed above. It may be obstacles 16, such as pillars 17, debris or any other artefact of the at least one zone 12, 12', 12", By applying a transformation on the information detected by the detector and/or on identified tracks 41', 41", 41‴ for mapping tracks of candidate biological cells 4, 4', 4", 4‴ not belonging to an associated biological cell category of the zone 12, 12', 12" onto straight paths through the flow channel, movements around obstacles may be easier to analyze.

The processor 26 may be configured to normalize the transit times for each candidate biological cell 4, 4', 4", 4‴ and for each segment 18. This normalization would allow for a simple way of analyzing flow channels 10 of different shapes, or for channels 10 having obstacles. It may further allow for a compensation for candidate biological cells 4, 4', 4", 4‴ being stuck during the flow through the at least one zone 12, 12', 12". From this, the processor 26 may divide the zone into time segments 182 for the further analysis.

The processor 26 may be configured to perform a weighted analysis of the transit times of each segment 18. The segments 18 may be then given different weights in the further analysis. Thus, the transit times of each segment 18 may be analyzed and based on that, decide if any segment 18 is of higher or lower importance. For instance, if the CMM surface 14 is covering the pillars or other obstacles, it may be of more interest to analyze the segments 18 covering such pillars. Likewise, knowing that a part of a zone 12, 12', 12", for instance before or after a pillar, may give rise to abnormal movements, may make it of less interest to analyze and therefore, that segment 18 may be given less weight in the analysis.

The processor 26 may be configured to divide each zone 12, 12', 12" into individual segments 18 for each of the candidate biological cells 4, 4', 4", 4'", depending on the path taken by the biological cell. Thus, candidate biological cells 4, 4', 4", 4‴ taking different paths though the at least one zone 12, 12', 12" may have passed a different number of segments 18 and may have a different number of transit times.

Thus, candidate biological cells 4, 4', 4", 4‴ taking completely different paths through the zone 12, 12', 12" may still be comparable, when taking into account the different paths.

The processing of the information further comprises identifying candidate biological cells 4, as illustrated in Fig 4c where the candidate biological cells 4', 4" and 4‴ are identified. These may be identified in information at a single point in time from the at least one detector 20. The candidate biological cells 4 are identified from the information detected by the at least one detector 20. The candidate biological cells 4 may be biological cells 4 belonging to the cell category of the zone 12. For instance, the candidate biological cell 4' of Fig. 2b belong to the associated cell category with the first zone 12', while the candidate biological cell 4" belongs to the associated cell category of the second zone 12". The candidate biological cells 4 may be a biological cell 4 not belonging to the cell category of the zone 12. For instance, the candidate biological cell 4' does not associate with the cell category of zone 12". The candidate biological cell 4 may however also be non-cell 4‴. The non-cell 4‴ could be a debris in the flow of liquid, a dead or injured cell, an artefact in the CMM surface of the zone or an imaging artefact interpreted by the detector as a cell.

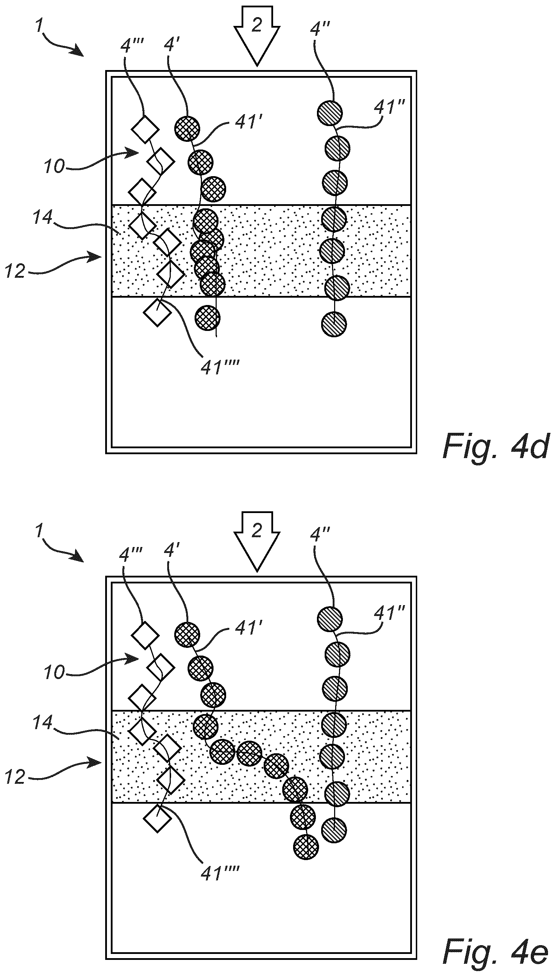

Further, the processor 26 is configured to identify tracks 41', 41", 41‴ from each of the candidate biological cells 4', 4", 4'". This is illustrated in Fig. 4d and 4e, illustrating how different candidate biological cells 4, 4', 4", 4" may move through the zone 12. The track 41' corresponds to a sequence of identified locations of the identified candidate biological cell 4' in sequential time points of the information from the at least one detector 20. The track 41" corresponds to a sequence of identified locations of the identified candidate biological cell 4". The track 41‴ corresponds to a sequence of identified locations of the identified candidate biological cell 4‴. Thus, each identified candidate biological cell 4', 4", 4‴ has its own track 41', 41", 41‴.

The tracking monitors the position of the detected candidate biological cells 4', 4", 4‴ in the flow channel 10 over a time-sequence during which the candidate biological cell 4', 4", 4‴ passes the zone 12. Thus, the movements of the each of the candidate biological cell 4', 4", 4‴ passing the zone 12 is represented by the tracks 41', 41", 41‴.

For each track 41', 41", 41‴ of the candidate biological cells 4', 4", 4‴ a set of transit sites are determined. Each transit time represents the time it takes for one candidate biological cell 4', 4", 4‴ to pass through the respective segment 18. Thus, the set of transit times represents the time it takes for the candidate biological cell 4', 4", 4‴ to pass through the zone 12.

A candidate biological cell 4', 4", 4‴ belonging to the cell category associated with the zone 12 may have longer transit times than a candidate biological cell 4', 4", 4‴ not belonging to the cell category associated to the zone 12. The candidate biological cell 4', 4", 4‴ belonging to the cell category associated with the zone 12 interacts with the CMM surface 14 such that the passage of the candidate biological cells 4', 4", 4‴ takes longer than for a candidate biological cell 4', 4", 4‴ not interacting with the CMM surface 14.

For each candidate biological cell 4', 4", 4‴, the set of transit times are processed to characterize the candidate biological cell 4', 4", 4‴.

The characterization may be used to determine if the candidate biological cell 4', 4", 4‴ belongs to the biological cell category associated with the zone 12.

The processing of the set of transit times may comprise determining at least one parameter representing a sequence of transit times within the set of transit times, and characterizing the candidate biological cell 4', 4", 4‴ based on the at least one parameter.

The determining at least one parameter representing a sequence of transit times may further comprise defining at least one subset of the at least one parameter and characterizing the candidate biological cell 4', 4", 4‴ based on the at least one subset.

The processing may involve investigating the distribution of the segment transit times. From the distribution, parameters such as mean transit time, standard deviation of all transit times of candidate biological cell 4', 4", 4‴, skewness of the distribution of all segment transit times may be determined.

The parameters from each of the candidate biological cells 4', 4", 4‴ may be compared to each other. For instance, the skewness of the transit times may be plotted against the standard deviation of the transit times. This may result in clusters, such that the candidate biological cells 4', 4", 4‴ belonging to the biological cell category associated with the zone 12 may have similar skewness and standard deviation of the transit times. Further, the candidate biological cells 4', 4", 4‴ not belonging to the biological cell category associated with the zone 12 may have a similar skewness and standard deviation of the transit times. The skewness and the standard deviation of the candidate biological cells 4', 4", 4‴ belonging to the biological cell category associated with the zone 12 may differ from the skewness and standard deviation of the candidate biological cells 4', 4", 4‴ not belonging to the biological cell category associated with the zone 12.

It should be understood that skewness and standard deviation is only two examples of descriptive parameters which may be used to classify the candidate biological cells 4', 4", 4‴ based on their transit time over the CMM surface 14.

The processing of the set of transit times may be made by use of a machine learning model.

The machine learning model may be based on supervised training. The machine learning model may be an artificial neural network. The machine learning model may thus be based on sets of transit times determined for known biological cells and/or other particles passing through the flow channel.

The machine learning model may be based on training for a particular flow channel or based on general training of cells passing over CMM surfaces.

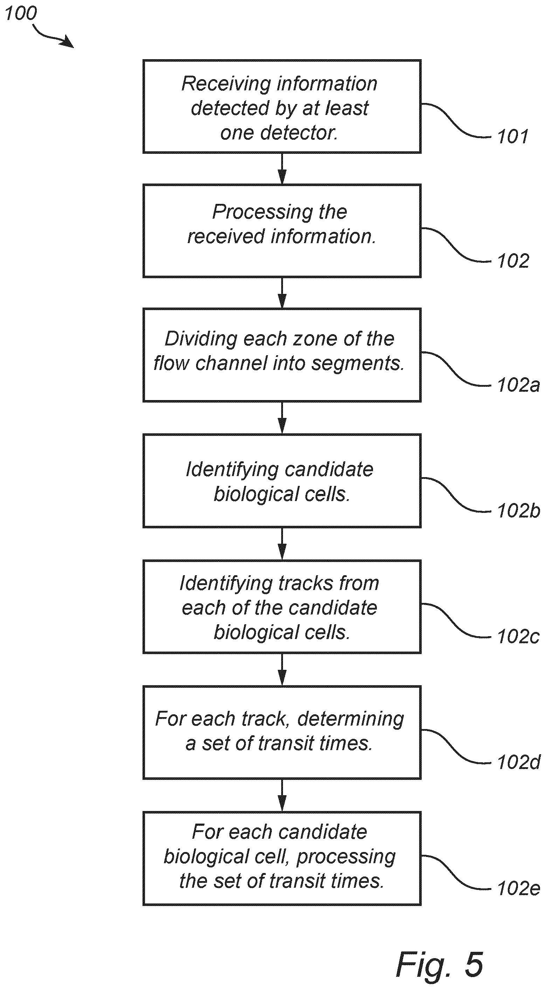

Fig. 5 illustrates a flow chart of a method 100 for characterizing biological cells 4', 4", 4‴.

The method 100 comprises receiving 101 information detected by at least one detector 20 representing biological cells 4', 4", 4‴, carried by a flow of liquid 2. The candidate biological cells 4', 4", 4‴ are to be characterized when passing at least one zone 12 in a flow channel 10.

Each zone 12 of the flow channel 10 is associated with a cell category. Each zone 12 of the flow channel 10 comprises at least one surface 14 coated with molecules having an affinity to the cell category associated with the zone. The at least one surface 14 of the zone 12 being configured to modify, by the molecules coating the at least one surface 14, a movement of a biological cell 4', 4", 4‴ belonging to the associated cell category of the zone as the biological cell 4', 4", 4‴ carried by the flow of liquid 2 passes the zone 12. The at least one surface 14 forms at least one biological cell movement modifying (CMM) surface. The modified movement of the biological cell 4', 4", 4‴ caused by the CMM surface forms a specific modified movement.

The method 100 further comprises processing 102 the received information. The processing 102 of the received information comprises dividing 102a each zone 12 of the flow channel 2 into segments 18. The segmentation of the at least one zone 12 is a computational process and it will not affect the candidate biological cells 4', 4", 4‴ passing the zone 12. The processor 26 may further be configured to divide the flow channel 10 into segments 18 outside of the zone 12 as well.

The processing 102 of the received information further comprises identifying 102b candidate biological cells 4', 4", 4‴ in the flow channel 10. These may be identified in information at a single point in time from the at least one detector 20. The candidate biological cells 4 are identified from the information detected by the at least one detector 20.

The processing 102 of the received information further comprises identifying 102c tracks 41', 41", 41‴ from each of the candidate biological cells 4', 4", 4‴. The tracking monitors the position of the detected candidate biological cells 4', 4", 4‴ in the flow channel 10 over a time-sequence during which the candidate biological cell 4', 4", 4‴ passes the zone 12. Thus, the movements of the candidate biological cell 4', 4", 4‴ passing the zone 12 is represented by the tracks 41', 41", 41‴.

For each track 41', 41", 41‴ of the candidate biological cells 4', 4", 4‴, the processing 102 of the received information further comprises determining 102d a set of transit times. Each transit time in the set represents a transit time of the candidate biological cell 4', 4", 4‴ through a segment 18. Each transit time represents the time it takes for one candidate biological cell 4', 4", 4‴ to pass through the respective segment 18. Thus, the set of transit times represents the time it takes for the candidate biological cell 4', 4", 4‴ to pass through the zone 12.

For each candidate biological cell 4', 4", 4‴, the processing 102 of the received information further comprises processing 102e the set of transit times for characterizing the candidate biological cell 4', 4", 4‴.

The processing 102e may be made by determining whether the candidate biological cell 4', 4", 4‴ is a biological cell 4', 4", 4‴ belonging to an associated biological cell category of each respective zone 12.

The processing 102e of the set of transit times may comprise determining at least one parameter representing a sequence of transit times within the set of transit times, and characterizing the candidate biological cell 4, 4', 4", 4‴ based on the at least one parameter.

The processing 102e may involve investigating the distribution of the segment transit times. From the distribution, parameters such as mean transit time, standard deviation of all transit times of candidate biological cell 4', 4", 4‴, skewness of the distribution of all segment transit times may be determined.

The parameters from each of the candidate biological cells 4', 4", 4‴ may be compared to each other. For instance, the skewness of the transit times may be plotted against the standard deviation of the transit times. This may result in clusters, such that the candidate biological cells 4', 4", 4‴ belonging to the biological cell category associated with the zone 12 may have similar skewness and standard deviation of the transit times. Further, the candidate biological cells 4', 4", 4‴ not belonging to the biological cell category associated with the zone 12 may have a similar skewness and standard deviation of the transit times. The skewness and the standard deviation of the candidate biological cells 4', 4", 4‴ belonging to the biological cell category associated with the zone 12 may differ from the skewness and standard deviation of the candidate biological cells 4', 4", 4‴ not belonging to the biological cell category associated with the zone 12.

It should be understood that skewness and standard deviation are only two examples of descriptive parameters which may be used to classify the candidate biological cells 4', 4", 4‴ based on their transit time over the CMM surface 14.

The processing 102e of the set of transit times may be made by use of a machine learning model.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (1) for characterizing biological cells (4, 4', 4", 4‴) comprising:
a flow channel (10) configured to pass a flow of liquid (2), wherein:
the flow of liquid (2) carries the biological cells (4, 4', 4", 4‴) to be characterized,
the flow channel (10) comprises at least one zone (12, 12', 12"),
each zone (12, 12', 12") of the flow channel (10) is associated with a cell category, and
each zone (12, 12', 12") of the flow channel (10) comprises at least one surface coated with molecules having an affinity and specificity to the cell category associated with the zone (12, 12', 12"), the at least one surface of the zone (12, 12', 12") being configured to modify, by the molecules coating the at least one surface, a movement of a biological cell (4, 4', 4", 4‴) belonging to an associated biological cell category of the zone (12, 12', 12") as the cell (4, 4', 4", 4‴), carried by the flow of liquid (2), passes the zone (12, 12', 12"), whereby the at least one surface forms at least one cell movement modifying (CMM) surface (14), and whereby the modified movement of the biological cell (4, 4', 4", 4‴) caused by the CMM surface (14) forms a specific modified movement;
at least one detector (20) configured to detect information representing the biological cells (4, 4', 4", 4‴), carried by the flow of liquid (2), passing the at least one zone (12);
a processor (26) configured to receive the information detected by the at least one detector (20), and to process the information, wherein processing of the information comprises:
dividing each zone (12, 12', 12") of the flow channel into segments (18);
identifying candidate biological cells (4, 4', 4", 4‴);
identifying tracks (41', 41", 41‴) from each of the candidate biological cells;
for each track (41', 41", 41‴) of the candidate biological cells (4, 4', 4", 4‴), determining a set of transit times, wherein each transit time in the set represents a transit time of the candidate biological cell (4, 4', 4", 4‴) through a segment (18);
for each candidate biological cell (4, 4', 4", 4‴), processing the set of transit times for characterizing the candidate biological cell (4, 4', 4", 4'").

2. A device (1) for characterizing biological cells (4, 4', 4", 4‴) according to claim 1, wherein the processing of the set of transit times comprises determining at least one parameter representing a sequence of transit times within the set of transit times, and characterizing the candidate biological cell (4, 4', 4", 4‴) based on the at least one parameter.

3. A device (1) for characterizing biological cells (4, 4', 4", 4‴) according to anyone of claims 1 to 2, wherein the processing of the set of transit times is made by use of a machine learning model.

4. A device (1) for characterizing biological cells (4, 4', 4", 4‴) according to anyone of claims 1 to 3, wherein the characterization of the candidate biological cells (4, 4', 4", 4‴) comprises a classification of the candidate biological cells (4, 4', 4", 4‴).

5. A device (1) for characterizing biological cells (4, 4', 4", 4‴) according to anyone of claims 1 to 4, wherein the processor (26) is configured to divide each zone (12) into at least 100 segments (18).

6. A device (1) for characterizing biological cells (4, 4', 4", 4‴) according to any one of claims 1 to 5, wherein the processor (26) is configured to divide the flow channel (10) into segments (18) outside of the at least one zone (12).

7. A device (1) for characterizing biological cells (4, 4', 4", 4‴) according to any one of claims 1 to 6, wherein the flow channel (10) comprises a plurality of zones (12, 12', 12"), each having an affinity and specificity to a separate cell category.

8. A device (1) for characterizing biological cells (4, 4', 4", 4‴) according to any one of claims 1 to 7, wherein the processor (26) is configured to divide each zone (12) into segments having a fixed and equal length (181).

9. A device (1) for characterizing biological cells (4, 4', 4", 4‴) according to any one of claims 1 to 8, wherein the processor (26) is configured to divide each zone (12) into segments (18, 181), each having a length of approximately 25 µm, such as between 20 - 30 µm.

10. A device (1) for characterizing biological cells (4, 4', 4", 4‴) according to any one of claims 1 to 8, wherein the processor (26) is configured to divide each zone (12) into segments having a fixed and equal transit time (182) for candidate biological cells (4, 4', 4", 4‴) whose movement is not modified by the CMM surface (14, 14', 14") of the zone (12, 12', 12").

11. A device (1) for characterizing biological cells (4, 4', 4", 4‴) according to any one of claims 1 to 10, wherein the processor (26) is configured to apply a transformation on the information detected by the detector and/or on identified tracks (41', 41", 41‴) for mapping tracks of candidate biological cells (4, 4', 4", 4‴) whose movement is not modified by the CMM surface (14, 14', 14") of the zone (12, 12', 12") onto (41', 41", 41‴) straight paths through a virtual flow channel.

12. A device (1) for characterizing biological cells (4, 4', 4", 4‴) according to any one of claims 1 to 11, wherein the detector (20) is an image sensor (24).

13. A device (1) for characterizing biological cells (4, 4', 4", 4‴) according to claim 12, wherein the device (1) further comprises:
a light source (22) configured to illuminate a biological cell (4, 4', 4", 4‴) in the flow channel (10) such that an interference pattern is formed by interference between light being scattered by the illuminated biological cell (4, 4', 4", 4‴) and non-scattered light from the light source (22); and
wherein the image sensor (24) configured to detect an image series that represents a time-sequence of interference patterns of the illuminated biological cell (4, 4', 4", 4‴).

14. A method (100) for characterizing biological cells (4, 4', 4", 4‴), the method (100) comprising:
receiving (101) information detected by at least one detector (20) representing biological cells (4, 4', 4", 4‴), carried by a flow of liquid (2), to be characterized passing at least one zone (12) in a flow channel (10), wherein
each zone (12) of the flow channel (10) is associated with a cell category,
each zone (12) of the flow channel (10) comprises at least one surface (14) coated with molecules having an affinity to the cell category associated with the zone, the at least one surface (14) of the zone (12) being configured to modify, by the molecules coating the at least one surface (14), a movement of a biological cell (4, 4', 4", 4‴) belonging to the associated cell category of the zone as the biological cell (4, 4', 4", 4‴) carried by the flow of liquid (2) passes the zone (12), whereby the at least one surface (14) forms at least one biological cell movement modifying (CMM) surface, and whereby the modified movement of the biological cell (4, 4', 4", 4‴) caused by the CMM surface forms a specific modified movement,
processing (102) the received information, wherein said processing (102) of the received information comprises:
dividing (102a) each zone (12) of the flow channel (2) into segments (18);
identifying (102b) candidate biological cells (4, 4', 4", 4‴) in the flow channel (10);
identifying (102c) tracks (41', 41", 41‴) from each of the candidate biological cells (4, 4', 4", 4‴);
for each track (41', 41", 41‴) of the candidate biological cells (4, 4', 4", 4‴), determining (102d) a set of transit times, wherein each transit time in the set represents a transit time of the candidate biological cell (4, 4', 4", 4‴) through a segment (18);
for each candidate biological cell (4, 4', 4", 4‴), processing (102e) the set of transit times for characterizing the candidate biological cell (4, 4', 4", 4'").

15. A computer program product (200) comprising computer-readable instructions such that when executed on a processor, the computer program product will cause the processor to perform the method (100) according to claim 14.
